Europäisches Patentamt

European Patent Office

Office européen des brevets

(11) Veröffentlichungsnummer: **0 106 837**
**B1**

(12) **EUROPÄISCHE PATENTSCHRIFT**

(45) Veröffentlichungstag der Patentschrift: 07.11.90

(21) Anmeldenummer: 83890169.2

(22) Anmeldetag: 26.09.83

(51) Int. Cl.⁵: **A 61 K 37/02,** A 61 K 39/395,
A 61 K 39/12

(54) **Verwendung von Peptiden.**

(30) Priorität: 05.10.82 AT 3674/82

(43) Veröffentlichungstag der Anmeldung:
25.04.84 Patentblatt 84/17

(45) Bekanntmachung des Hinweises auf die
Patenterteilung:
07.11.90 Patentblatt 90/45

(84) Benannte Vertragsstaaten:
AT BE CH DE FR GB IT LI NL SE

(56) Entgegenhaltungen:
FR-A-2 444 466
GB-A-1 267 591

INFECTION AND IMMUNITY, Band 37, Nr. 3,
September 1982, Seiten 869-874; F.X. HEINZ et
al.: "Monoclonal antibodies to the structural
glycoprotein of Tick-Borne Encephalitis Virus"

CHEMICAL ABSTRACTS, Band 99, Nr. 3, 18. Juli
1983, Seite 345, Zusammenfassung Nr. 19361j,
Columbus, Ohio, US; F.X. HEINZ et al.: "A
topological and functional model of epitopes on
the structural glycoprotein of tick-borne
encephalitis virus defined by monoclonal
antibodies"

(73) Patentinhaber: IMMUNO Aktiengesellschaft für
chemisch-medizinische Produkte
Industriestrasse 72
A-1220 Wien (AT)

(72) Erfinder: Heinz, Franz Xaver
Brühlerstrasse 114/9
A-2340 Mödling (AT)
Erfinder: Kunz, Christian, Prof.Dr.
Naaffgasse 71
A-1180 Wien (AT)

(74) Vertreter: Wolfram, Gustav, Dipl.-Ing.
Schwindgasse 7 P.O. Box 205
A-1041 Wien (AT)

(56) Entgegenhaltungen:
INFECTION AND IMMUNITY, Band 33, Nr. 1, Juli
1981, Seiten 250-257; F.X. HEINZ et al.:
"Antigenic and Immunogenic properties of
defined physical forms tick-borne encephalitis
virus structural proteins"

**Beschreibung**

Die Erfindung betrifft die Verwendung von Peptiden und peptidhältigen Verbindungen, enthaltend eine oder mehrere Antigendeterminante(n) des FSME-Virus, zur Herstellung von gegen FSME-Virusinfektionen wirksamen Vakzinen.

Die Frühsommermeningeoenzephalitis (FSME) kommt in weiten Teilen Europas, vor allem Mittel- und Osteuropa, sowie in Rußland vor und ist in diesen Ländern aufgrund der Erkrankungshäufigkeit und der Schwere der Erkrankung von großer humanmedizinischer Bedeutung. Der Erreger, das FSME-Virus, wird in den allermeisten Fällen durch den Biß einer infizierten Zecke übertragen und gehört zur Gruppe der Flaviviren. Das FSME-Virus besitzt drei Strukturproteine (E, C, M), von denen jedoch nur das Glykoprotein (E) protektive Immunantwort nach Infektion oder aktiver Immunisierung induziert (Infection and Immunity, July 1981, Vol. 33, No. 1 (250—257), "Antigenic and Immunogenic Properties of Defined Physical Forms of Tick-Borne Encephalitis Virus Structural Proteins", F. X. Heinz et al.).

Die Immunantwort gegen Proteinantigene ist üblicherweise heterogen, d.h. es werden gegen verschiedene Antigendeterminanten des Glykoproteins gerichtete Antikörper induziert, von denen ein Teil für die protektive Wirksamkeit verantworlich ist. Es können jedoch auch Antikörper induziert werden, die selber keine Schutzwirkung besitzen, aber die Bindung protektiver Antikörper an das Virus blockieren und dadurch deren Schutzwirkung herabsetzen.

Die Erfindung bezweckt die Bereitstellung von Vakzinen, die antigenreaktive Peptide enthalten, die protektive Antikörper induzieren, vorzugsweise aber möglichst frei sind von Sequenzen, die mit blockierenden Antikörpern reagieren.

Es wurde gefunden, daß das FSME-Virus-Glykoprotein eine Mehrzahl von Antigendeterminanten aufweist, von denen vier durch ihre Reaktivität mit monoklonalen Antikörpen gekennzeichnet sind, welche Antikörper definiert sind durch

a) protektive Wirkung im passiven Mäuseschutzversuch und/oder

b) virusneutralisierende Wirkung im Neutralisationstest. Der passive Mäueschutzversuch und der Neutralisationstest sind in der Literatur beschrieben, u.zw. u.a. in der bereits genannten Literaturstelle von Heinz et al., 1981.

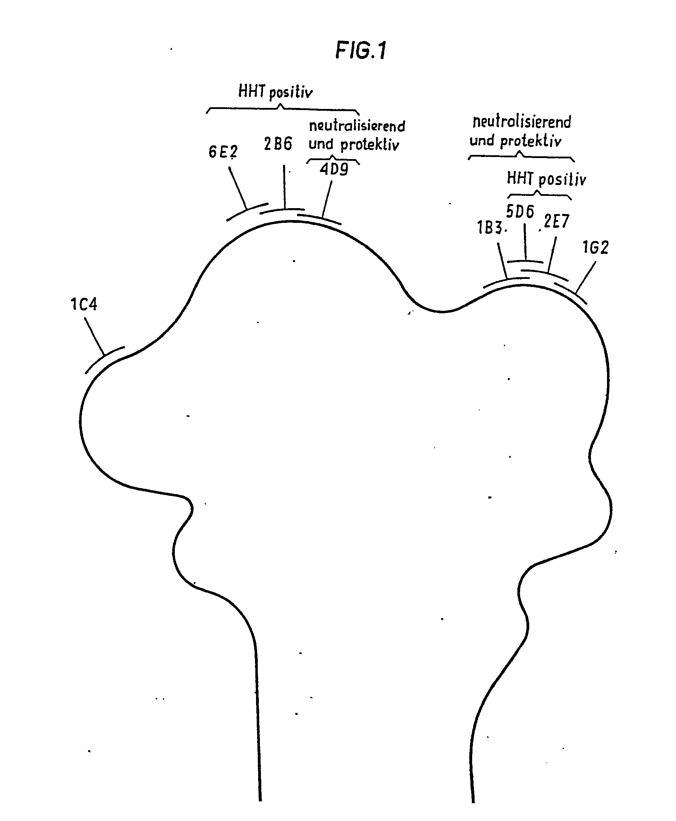

In Fig. 1 ist das Struktur-Glykoproteinmolekül durch eine Wellenlinie wiedergegeben. Die topographische Verteilung der verschiedenen Antigendeterminanten wird durch die Bindungsstellen der entsprechenden, mit ⊥-Symbolen bezeichneten monoklonalen Antikörper veranschaulicht, wobei eine Überlappung des Bondenstriches eine gegenseitige Hemm- bzw. Blockierungswirkung der Antikörper bedeutet. Antikörper, die im passiven Mäuseschutzversuch eine protektive Wirkung haben, sind mit "protektiv", jene, die im Neutralisationstest eine virusneutralisierende Wirkung haben, mit "neutralisierend" bezeichnet. Antikörper, die im Hämagglutinationshemmungstest eine positive Reaktion zeigen, sind mit "HHT positiv" bezeichnet.

Im einzelnen sind in dieser Darstellung die Antikörper 4D9, 1B3, 5D6 und 2E7 protektiv und neutralisierend.

Dementsprechend besteht die Erfindung in der Verwendung von Peptiden entsprechend einer Teilaminosäuresequenz des FSME-Virus-Glykoproteins E zur Herstellung von gegen FSME-Virusinfektionen wirksamen Vakzinen, enthaltend eine oder mehrere Antigendeterminanten des FSME-Virus, gekennzeichnet durch ihre Reaktivität gegenüber einem der monoklonalen Antikörper 4D9, 1B3, 5D6, 2E7 gemäß Fig. 1, die definiert sind durch

a) protective Wirkung im passiven Mäuseschutzversuch und/oder

b) virusneutralisierende Wirkung im Neutralisationstest.

Weitere Ausführungsformen der Erfindung bestehen darin, daß als antigenreaktive Peptide solche verwendet werden, die eine Reaktivität gegenüber dem Antikörper 1B3 zeigen, wobei dieser Antikörper zusätzlich zu den oben angeführten Eigenschaften die weiteren Merkmale

c) eine fehlende oder sehr niedrige Aktivität im Hämagglutinationshemmungstest,

d) gleiche Reaktivität im Enzymimmunassay mit FSME-Virus, dem fernöstlichen Subtyp des FSME-Virus (Sofyn) und Louping III Virus; fehlende Reaktivität mit West Nile und Murray Valley Encephalitis Virus, und

e) Hemmung der Bindung monoklonaler Antikörper der Charakteristik 2E7 und 5D6 (Fig. 1) an das Virus gemessen im kompetitiven Radioimmunassay aufweist;

daß als antigenreaktive Peptide solche verwendet werden, die eine Reaktivität gegenüber dem Antikörper 4D9 zeigen, wobei dieser Antikörper zusätzlich zu den oben angefuhrten Eigenschaften die weiteren Merkmale

f) eine hämagglutinationshemmende Aktivität,

g) gleiche Reaktivität im Enzymimmunassay mit FSME-Virus und Louping III Virus, verminderte Reaktivität mit dem fernöstlichen Subtyp des FSME-Virus (Sofyn), fehlende Reaktivität mit West Nile und Murray Valley Encephalitis Virus sowie

h) Hemmung der Bindung monoklonaler Antikörper der Charakteristik 2B6 (Fig. 1) an das Virus gemessen im kompetitiven Radioimmunassay aufweist;

daß als antigenreaktive Peptide solche verwendet werden, die eine Reaktivität gegenüber dem Antikörper 2E7 zeigen, wobei dieser Antikörper zusätlich zu den oben angeführten Eigenschaften die weiteren Merkmale

f) eine hämagglutinationshemmende Aktivität,

d) gleiche Reaktivität im Enzymimmunassay mit FSME-Virus, dem fernöstlichen Subtyp des FSME-Virus (Sofyn) und Louping III Virus; fehlende Reaktivität mit West Nile und Murray Valley Encephalitis Virus, sowie

i) Hemmung der Bindung monoklonaler Antikörper der Charakteristik 5D6, 1B3 und 1G2 (Fig. 1), gemessen im kompetitiven Radioimmunassay, aufweist;

daß als antigenreaktive Peptide solche verwendet werden, die eine Reaktivität gegenüber dem Antikörper 5D6 zeigen, wobei dieser Antikörper zusätlich zu den oben angeführten Eigenschaften die weiteren Merkmale

f) eine hämagglutinationshemmende Aktivität,

d) gleiche Reaktivität im Enzymimmunassay mit FSME-Virus, dem fernöstlichen Subtyp des FSME-Virus (Sofyn) und Louping III Virus; fehlende Reaktivität mit West Nile und Murray Valley Encephalitis Virus, sowie

k) Hemmung der Bindung monoklonaler Antikörper der Charakteristik 1B3 und 2E7 (Fig. 1), gemessen im kompetitiven Radioimmunassay, aufweist.

Aus der Darstellung in Fig. 1 ist weiters ersichtlich, aß es erwünscht ist, antigenreaktive Peptide vorzusehen, die frei sind von Sequenzen, die mit hemmenden bzw. blockierenden Antikörpern reagieren; solche blockierende Antikörper sind die mit 2B6 und 1G2 bezeichneten.

Dementsprechend besteht eine bevorzugt Ausführungsform der Erfindung darin, daß als antigene Peptide solche verwendet werden, die zusätzlich zu den oben angeführten Eigenschaften die Bedingung erfüllen, daß sie frei sind von Sequenzen, die mit blockierenden monoklonalen Antikörpern 2B6 bzw. 1G2 (Fig. 1) folgender Charakteristik reagieren, wobei der monoklonale Antikörper 2B6 definiert ist durch

l) Hemmung der Bindung des protektiven monoklonalen Antikörpers 4D9 sowie des nichtprotektiven monoklonalen Antikörpers 6E2, gemessen im kompetitiven Radioimmunassay,

m) fehlende oder sehr geringe Aktivität im passiven Mäuseschutzversuch,

n) fehlende oder sehr niedrige Aktivität im Neutralisationstest,

f) hämagglutinationshemmende Aktivität,

d) gleiche Reaktivitätim Enzymimmunsassy mit FSME-Virus, dem fernöstlichen Subtyp des FSME-Virus (Sofyn) und Louping III Virus, abgeschwächte Reaktivität mit West Nile und Murray Valley Encephalitis Virus, und wobei der monoklonale Antikörper 1G2 definiert ist durch

o) Hemmung der Bindung protektiver monoklonaler Antikörper des Charakteristik 2E7, gemessen im kompetitiven Radioimmunassay,

m) fehlende oder sehr geringe Aktivität im passiven Mäuseschutzversuch,

n) fehlende oder sehr niedrige Aktivität im Neutralisationstest,

c) fehlende oder sehr geringe Aktivität im Hämagglutinationshemmungstest,

p) gleiche Reaktivität im Enzymimmunassay mit FSME-Virus und dem fernöstlichen Subtyp des FSME-Virus (Sofyn), abgeschwächte Reaktivität mit Louping III Virus, fehlende Reaktivität mit West Nile und Murray Valley Encephalitis Virus.

Die Reagenzien zur Identifizierung von Antigendeterminaten enthalten monoklonale Antikörper mit den angegebenen Eigenschaften. Ihre Herstellung erfolgt nach an sich bekannten Verfahren dadurch, daß Milzzellen von gegen FSME-Virus immunisierten Mäusen mit ausmyelommzellen zu Hybridzellen (Hybridome) fusioniert, die Hybridzellen zu Zellklonen weiter gezüchtet und die sezernierten Antikörper nach Prüfung ihrer Reaktivität isoliert werden. Dieses Verfahren ist beispielsweise in Nature (London) Vol. 256, 1975, 495—497, "Continuous Cultures of Fused Cells Secreting Antibody of Predefined Specificity", Köhler et al. beschrieben. Demgemäß werden Mäuse mit dem Glykoprotein immunisiert und die Milzzellen dieser Tiere mit Maus-Myelomzellen in der Weise verschmolzen, daß die resultierenden Hybridzellen (Hybridome) Antikörper zezernieren, die mit den Glykoprotein reagieren und zur Produktion dieser Antikörper in vitro und in vivo vermehrt werden können. Durch Klonen dieser Hybridome werden Zellpopulationen erhalten, die nur eine Art vollkommen identischer Antikörpermoleküle sezernieren. Diese monoklonalen Antikörper stellen hochspezifische Reagenzien dar, die genau umgrenzte Antigendeterminanten am Glykoprotein und deren Funktion charakterisieren Durch Analyse der Reaktivität dieser Antikörper mit serologisch verwandten Virusvarianten sowie durch funktionelle Tests (Hämagglutinationshemmungstest, Neutralisationstest, passivier Mäusechutzversuch) werden solche Antikörper selektioniert sowie deren Funktionen bestimmt, die mit unterschiedlichen Antigendeterminanten reagieren. Mit Hilfe eines kompetitiven Radioimmunassays wird die topographische Verteilung der durch die monoklonalen Antikörper definierten Antigendeterminanten ermittelt und festgestellt, welche Antikörper die Bindung eines anderen Antikörpers blockieren.

Im folgenden werden die Herstellung der monoklonalen Antikörper und die Durchführung der verschiedenen Tests, die zur Charakterisierung der monoklonalen Antikörper und im weiterer Folge zur Bestimmung der antigenreaktiven Peptide dienen, im enzelnen beschrieben.

Herstellung der monokonalen Antikörper:

Zur Herstellung monoklonaler Antikörper werden 2 Monate alte Balb/c Mäuse mit dem nach Detergens-Solubilisierung durch Dichtegradienten-Zentrifugation isolierten Struktur-Glykoprotein des FSME-Virus (J. Gen. Virol. *49*, 125—132, F. X. Heinz et al., 1980, Formation of polymeric glycoprotein complexes from a flavivirus: Tick-borne encephalitis virus) intraperitoneal immunisiert. Den immunisierten Mäusen wird die Milz entnommen und in eine Zellsuspension übergeführt. Diese Milzzellen werden sodann nach einem bekannten Verfahren (Köhler et al., 1975, Nature (London) *256*, 495—497, Continuous cultures of fused cells secreting antibody of pre-defined specificity) mit Maus-Myelomzellen fusioniert, die einen Defekt im Enzym Hypoxanthinphosphoribosyltransferase (HGPRT) haben und daher in einem Medium enthaltend *H*ypoxanthin, *A*minopterin und *T*hymidin (HAT) absterben. Durch dieses Verfahren werden Hybridzellen (Hybridome) gewonnen, die spezifische, glykoproteinreaktive Antikörper sezernieren.

Zur Fusion werden etwa $1 \times 10^8$ Milzzellen mit $1 \times 10^7$ Maus-Myelomzellen in Gegenwart von 50% Polyäthylenglykol 4000 emischt und 90 Sekunden bei 37°C inkubiert. Daraufhin werden die Zellen in HAT-Medium suspendiert, auf Gewebekulturgefäße verteilt und bei 37°C inkubiert. Nur Hybride zwischen Myelom- und Milzzellen überleben in HAT-Medium, wobei die Fähigkeit, in vitro zu wachsen, von den Myelomzellen stammt und die Milzzellen das funktionsfähige Enzym HGPRT zur Verfügung stellen.

Durchführung des passiven Mäuseschutzversuches:

Die antikörperhältige Probe wird in den Verdünnungen 1:4, 16, 64 und 256 in phosphatgepufferter Salzlösung, pH 7,2, 15 g schweren Mäusen subcutan injiziert. Pro Verdünnung werden 10 Mäuse verwendet, die Dosis pro Maus beträgt 0,2 ml. Nach 24 Stunden werden die Mäuse mit FSME-Virus, enthaltend 100 bis 1000 letale Dosen $_{50}$ ($LD_{50}$ = jene Dosis, bei der die 50% der infizierten Tiere sterben) infiziert. Die Mäuse werden 3 Wochen lang beobachtet und die Ergebnisse nach der Methode von Reed et al. (1938, A simple method of estimating fifty per cent endpoints. Amer. J. Hyg. *27*, 493—497) berechnet. Der Titer im passiven Mäuseschutzversuch ist der reziproke Wert jener Verdünnung, bei der 50% der Tiere die Infektion mit der sonst tödlichen Dosis FSME-Virus überlebten.

Durchführung des Neutralisationstests (NT):

Für diesen Test wird eine Permanente Schweinenieren-Zellinie (PS 1) verwendet. Die antikörperhältige Probe wird in den Verdünnungen 1:30, 100, 300, 1000, 3000, 10.000, 30.000 und 100.000 in Minimum Essential Medium (MEM) enthaltend 10% foetales Kälberserum verdünnt und mit 100 infektiösen Dosen FSME-Virus 90 Minuten bei 37°C inkubiert. Je 50 μl diese Gemisches werden in die Vertiefungen von Gewebekultur-Mikrotiterplatten gegeben und mit je 100 μl PS 1-Zellen enthaltend $1 \times 10^5$ Zellen in MEM + 10% foetales Kälberserum versetzt. Nach 4 Tagen Inkubation bei 37°C wird der NT abgelesen. Der Titer im NT ist der reziproke Wert jener höchsten Antikörperverdünnung, bei der noch kein cytopathischer Effekt auftritt.

Durchführung des Hämagglutinationshemmungstests (HHT)

(nach Clarke et al., 1958, AMer. J. Trop. Med. Hyg. *7*, 561—573, Techniques for haemagglutination and haemagglutination inhibition with arthropod-borne viruses) Zur Entfernung unspezifischer Inhibitoren werden 0,2 ml antikörperhältige Probe mit 24 ml Aceton versetzt, 5 Minuten im Eisbad stehengelassen, worauf das gebildete Präzipitat abzentrifugiert wird. Das Sediment wird nochmals in 24 ml Aceton aufgenommen, 5 Minuten im Eisbad stehengelassen und wieder zentifugiert. Daraufhin wird das Sediment getrocknet und in 2 ml boratgepufferter Salzlösung pH 9,0 resuspendiert und mit Gänseerythrozyten zur Entfernung von Autoagglutininen 20 Minuten im Eisbad inkubiert. Die Erythrozyten werden sodann durch Zentrifugation entfernt und der Überstand stellt die 1:10 verdünnte abgesättigte Antikörperpräparation dar. Diese wird in Zweierstufen in boratgepufferter Salzlösung, pH 9,0, verdünnt und 0,2 ml jeder Verdünnungsstufe werden mit 0,2 ml FSME Virusantigen, enthaltend 4 hämagglutinierende Einheiten versetzt und 3 Stunden bei Raumtemperatur inkubiert. Danach werden 0,4 ml einer 0,25%igen Gänseerythrozytensuspension in Phosphatpuffer zugegeben, sodaß der pH-Wert des Gemisches 6,4 beträgt. Der Test wird bis zum Ablesen bei Raumtemperatur stehengelassen. Der Titer im HHT stellt den reziproken Wert jener höchsten Antikörperverdünnung dar, bei der die Hämagglutination der Gänseerythrozyten durch das FSME-Virus-Antigen noch gehemmt wird.

Durchführung des Enzymimmunassays:

Polystyrol-Mikrotiterplatten werden durch Inkubation (24 Stunden, Raumtemperatur) mit 50 μl pro Vertiefung gereinigtem FSME-Virus in einer Konzentration von 2 μg/ml in Carbonatpuffer, pH 9,6, beschichtet. Diese Mikrotiterplatten werden mit 2% Schafserum in phosphatgepufferter Salzlösung (PBS), pH, 7,4, abgesättigt (1 Stunde, 37°C) und sodann mit den Kulturüberständen (50 μl pro Vertiefung) 1 Stunde bei 37°C inkubiert. Daraufhin wird 3 mal mit PBS, pH 7,4, gewaschen und pro Vertiefung mit 50 μl Meerrettich-Peroxidase — konjugiertem Kaninchen — anti-Maus-Immunglobulin in PBS, pH 7,4, enthaltend 2% Schafserum und 2% Tween 20 1 Stunde bei 37°C inkubiert. Nach dreimaligem Waschem mit PBS, pH 7,4, werden 50 μl des Enzymsubstrates zugegeben (o-Phenylendiamin, 1 mg/ml in Phosphat-Citrat-Puffer, pH 5,0 enthaltend 1 μl Perhydrol/ml). Die Enzymreaktion wird nach 30 Minuten bei Raumtemperatur durch Zugabe von 100 μl 2N $H_2SO_4$ gestoppt und das Ausmaß der Farbreaktion mit Hilfe eines Vielkanal-Photometers bei einer Wellenlänge von 492 nm bestimmt.

Durchführung des kompetitiven Radioimmunassays:

Polystyrolkugeln (Durchmesser 6,4 mm, Precision Ball Co., Chicago, Ill.) werden mit gereinigtem FSME-Virus (2 µg/ml in Carbonatpuffer, pH 9,6) durch Inkubation bei 4°C über Nacht beschichtet und sodann durch Inkubation mit 2% Schafserum in PBS, pH 7,4 abgesättigt (37°C/1 Stunde). Die monoklonalen Antikörper werden in PBS, pH 7,4, enthaltend 2% Schafserum und 2% Tween 20 von $10^{-1}$ bis $10^{-6}$ verdünnt. Je 100 µl dieser Verdünnungen werden mit 100 µl des mit $^{125}$J nach der Methode von Markwell et al. (1978, Biochemistry 17, 4807—4817, Surface-Specific Iodination of Membrane Proteins of Viruses and Eucaryotic Cells Using 1,3,4,6-Tetrachloro-3α,6α-diphenylglycoluril) markierten Antikörpers enthaltend 100.000 counts/min in einem Teströhrchen gemischt. Zu jeder dieser Mischungen wird eine virusbeschichtete und abgesättigte Polystyrolkugel gegeben und bei 37°C eine Stunde inkubiert. Danach wird das Antikörpergemisch abgesaugt, die Kugeln werden noch viermal mit Leitungswasser gewaschen und sodann in einem δ-Spectrometer gemessen. In Abwesenheit eines blockierenden Antikörpers bindet der $^{125}$J-markierte Antikörper an die antigenbeschichtete Polystyrolkugel und markiert diese radioaktiv. Bei gleichzeitiger Inkubation mit einem nichtmarkierten monoklonalen Antikörper zeigt eine Herabsetzung der gebundenen Radioaktivität eine Bindung an eng benachbarte oder überlappende Epitope oder eine allo allosterische Wechselwirkung an. Wird die gebundene Radioaktivität nicht herabgesetzt, bedeutet dies, daß beide Antikörper an räumlich getrennte, unabhängige Antigendeterminanten binden.

Durch Anwendung der beschriebenen Tests können jene Aminosäuresequenzen des Glykoproteins identifiziert werden, die zur Induktion einer protektiven Immunantwort geeignet sin und somit als Bestandteil einer Vakzine gegen FSME verwendet werden können.

Beispiel: Nachdem — wie oben beschrieben — Hybridome, die glykoproteinspezifische Antikörper sezernieren, bereitgestellt wurden, wurde eine Analyse der Kulturüberstände mittels eines Enzymimmunassays durchgeführt, wobei eine positive Farbreaktion das Vorhandensein spezifischer, von den Hybridomen freigesetzter Antikörper anzeige.

Jene Hybridome, die FSME-Virus glykoproteinspezifische Antikörper sezernierten, wurden zur Klonierung einer limitierten Verdünnung unterworfen und solche Klone ausgewählt, welche die Nachkommmenschaft einer einzigen Zelle darstellten, sodaß die freigesetzten Antikörper tatsächlich monoklonal, d. h. in ihrem chemischen Aufbau vollkommen identisch sind und somit hochspezifische Reagenzien darstellen, die mit nur einem einzigen, durch diesen Antikörper genau definierten Epitop (= Antikörperbindungsstelle) am FSME-Virus-Glykoprotein reagieren.

Zur Aufklärung der Antigenstruktur dieses Glykoproteins wurde nun untersucht, welche monoklonalen Antikörper mit unterschiedlichen Antigendeterminanten am Glykoprotein reagierten, sowie welche Funktion und topographische Lage die enzelnen Determinanten hatten. Zu diesem Zweck wurde zunächst ene Variantenanalyse durchgeführt, wobei die Reaktivität der monoklonalen Antikörper mit dem FSME-Virus sowie mit serologisch näher und entfernter verwandten Flaviviren untersucht wure. Für diese Analyse wurde wieder der beschriebene Enzymimmunassay verwendet, wobei hochgereinigte Präparationen des FSME-Virus, des Fernöstlichen Subtyps des FSME-Virus (Sofyn), des Louping III Virus, des West Nile Virus und des Murray Valley Encephalitis Virus als Antigen verwendet wurden.

Die Ergebnisse sind in Tabelle 1 zusammengefaßt. Daraus geht hervor, daß einzelne monokonale Antikörper unterschiedliche Reaktivitätsmuster mit den verwendeten Virusvarianten zeigten und daher mit unterschiedlichen Antikörperbindungsstellen am Glykoprotein reagierten.

Die weitere Differenzierung und Charakterisierung wurde mit Hilfe des Hämagglutinationshemmungstests, des Neutralisationstests und des passiven Mäuseschutzversuches vorgenommen, deren Ergebnisse in Tabelle 2 wiedergegeben sind. In Ergänzung zur Variantenanalyse erlaubten diese Tests, monoklonale Antikörper, die an verschiedene Epitope binden, aufgrund funktioneller Kriterien zu differenzieren. Wie aus Tabelle 2 hervorgeht, wurden protektive und virusneutralisierende Antikörper von jenen Antigendeterminanten induziert, die durch die monoklonalon Antikörper 1B3, 4D9, 2E7 und 5D6 charakterisiert sind (Fig. 1).

Zur Aufklärung der räumlichen Verteilung jener Antigendeterminanten auf dem FSME-Virus-Glykoprotein, die mit den beschriebenen monoklonalen Antikörpern reagieren, sowie deren gegenseitige Beziehung, wurde in einem Radioimmunassay untersucht, ob die Bindung eines vorher radioaktiv markiertn Antikörpers durch nichtmarkierte Antikörper blockiert wird. Eine Herabsetzung der Bindung des radioaktiven Antikörpers durch den zweiten Antikörper zeigt eine enge Nachbarschaft oder allosterische Wechselwirkung der durch dieses Paar monoklonaler Antikörper definierten Epitope an.

Die Ergebnisse dieser Blockierungsversuche mit den vorher charakterisierten monoklonalen Antikörpern sind in Tabelle 3 zusammenfassend dargestellt.

Aufgrund der beschriebenen Untersuchungen war es möglich, die topographische Verteilung und die Funktion von unabhängigen oder überlappenden Epitopen am Glykoprotein des FSME-Virus in einem Modell darzustellen (Fig. 1). Diese Epitope stellen eng umschriebene Regionen des Proteins dar, die nur aus etwa 6 bis 7 Aminosäuren bestehen und durch ihre Reaktivität mit den ihrerseits chemisch genau definierten, homogenen, monoklonalen Antikörpern definiert sind.

Wie aus den auf dem Modell (Fig. 1) zusammengefaßten Ergebnissen hervorgeht, definieren die monoklonalen Antikörper 4D9, 1B3, 5D6 und 2E7 solche Antigendeterminanten, die virusneutralisierende und protektive Antikörper induzieren und daher die entscheidenden Bestandteile einer Vakzine darstellen.

Die durch 2B6 und 1G2 definierten Epitope können die Bildung solcher Antikörper induzieren, die selbst keine virusneutralisierende oder protektive Wirkung besitzen, jedoch die Bindung von schützenden Antikörpern blockieren und damit deren Wirksamkeit herabsetzen können. Vorzugsweise sollten solche Anteile nicht in einer Vakzine enthalten sein.

Die Erfindung umfaßt die Bereitstellung von Vakzinen, die antigenreaktive Peptide enthalten. Eine Moglickeit für die Herstellung von antigenreaktiven Peptiden, die für die erfindungsgemäße Verwendung in Frage kommen, ist die Fragmentierung des Glykoproteins durch enzymatische oder chemische Maßnahmen oder durch synthetischen Aufbau der entsprechenden Aminosäuresequenzen. Solche Sequenzen sind durch ihre Reaktivität mit den protektiven Antikörpern 1B3, 4D9, 2E7 und 5D6 definiert. Eine Möglichkeit der enzymatischen Fragmentierung besteht in der Umsetzung des Glykoproteins mit Proteasen, wie Trypsin und α-Chymotrypsin, wobei das Glykoproteinmolekül an bestimmten Stellen, die durch die Proteasen definiert sind, in Peptide gespalten werden. Die gewonnenen Spaltstücke enthalten reaktive Peptide, die mit den protektiven monoklonalen Antikörpern 1B3, 4D9, 2E7 und 5D6 reagieren. Die Proteasebehandlung wurde in folgender Weise vorgenommen.

Proteasebehandlung:

Das Glykoprotein des FSME-Virus wurde durch Behandlung des gereinigten FSME-Virus mit Poly (9—10) oxyäthylen p-tert. octyl phenol und anschließende Dichtegradientenzentrifugation, wie von Heinz et al. (1980) beschrieben, isoliert. Diese Glykoptoteinlösung (40 µg/ml) in 0,05 M Triäthanolaminpuffer, 0.1 M NaCl, pH 8,0 wurde mit Trypsin oder α-Chymotrypsin versetzt, sodaß das Verhältnis Glykoprotein: Protease 50: 1 betrug. Die Proteaseverdaung wurde 24 Stunden bei 37°C durchgeführt. Als Kontrolle wurde Rinderalbumin unter vollkommen identischen Bedingungen der Proteasebehandlung unterzogen.

Der Nachweise der immunreaktiven Peptide erfolgte durch einen Blocking-Enzymimmunassay, der in folgender Weise vorgenommen wird. Das Prinzip besteht darin, daß die Bindung monoklonaler Antiköper an das Glykoprotein durch eine positive Farbreaktion angezeigt wird. Inkubiert man den Antikörper jedoch vorher mit einer Substanz, die spezifisch mit dem Antikörperr reagiert, wird dieser blockiert und kann nicht mehr an das Virusantigen binden. Eine Herbsetzung der Farbreaktion zeigt also das Vorliegen einer immunreaktiven Substanz an, die spezifisch mit dem untersuchten monoklonalen Antikörper reagiert.

Die Durchführung erfolgte im einzelnen in der Weise, daß proteasebehandeltes Glykoproein sowie Rinderalbumin (Kontrolle) in PBS, pH 7,4, enthaltend 2% Schafserum und 2% Tween 20 1:3, 10, 30, 100, 300 und 1000 verdünnt wird. Ein Teil dieser Verdünnungen wurde mit einem gleichen Teil des monoklonalen Antikörpers versetzt, der so verdünnt wurde, daß die durch ihn bewirkte positive Farbreaktion im Enzymimmunassay etwa eine Extinktionseinheit beträgt. Das Gemisch wurde eine Stunde bei 37°C inkubiert und dann in die Vertiefungen von virusbeschichteten Mikrotiterplatten zur Durchführung des beschriebenen Enzymimmunassays gegeben.

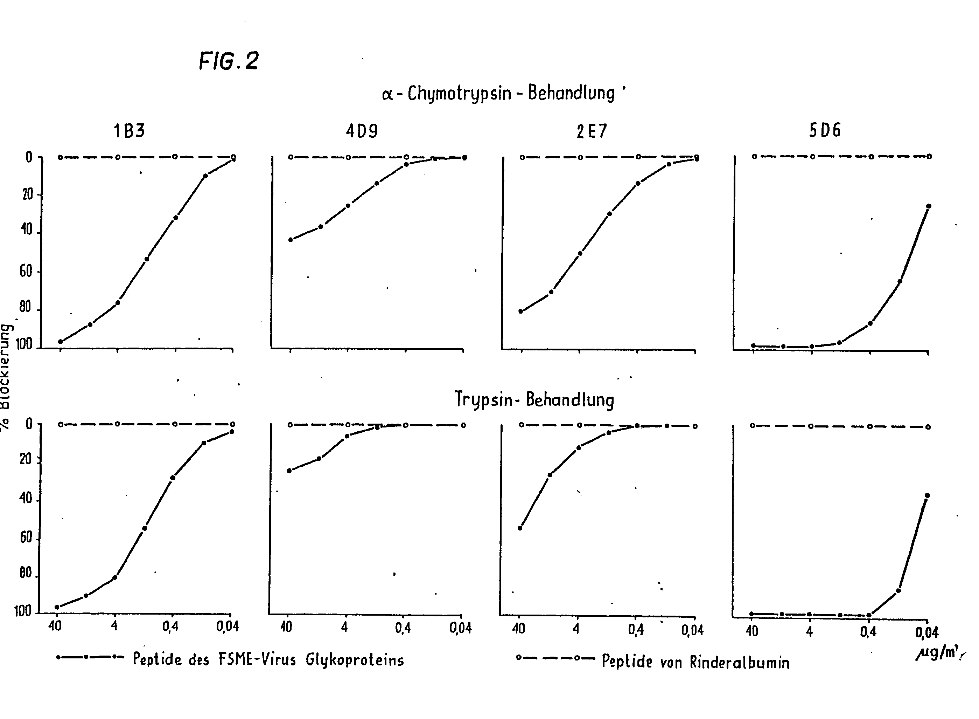

Die Ergebnisse eines solchen Enzymimmunassays sind aus Fig. 2 ersichtlich, worin die Blockierung der Bindung von protektiven monoklonalen Antikörpern an das FSME-Virus durch spezifische Reaktion mit Peptiden, die nach α-Chymotrypsin und Trypsin-Behandlung des Struktur-Glykoproteins erhalten wurden, dargestellt ist. In allen Fällen wurde das Vorhandensein von Peptiden nachgewiesen, die mit den schützenden monoklonalen Antikörpern reagierten und daher solche immunreaktive Sequenzen des FSME-Virus-Glykoproteins enthielten, die zur Induktion einer protektiven Immunantwort geeignet sind.

In em folgenden Beispiel 1 wird die Spaltung des FSME-Virus-Glykoproteins mit nachfolgender Identifizierung der erhaltenen Peptide, die durch monoklonale Antikörper definierte Antigendeterminanten enthalten, beschrieben.

Beispiel 1

Enzymatische Spaltung

Eine Probe von FSME-Virus-Glykoprotein (40 µg/ml) wurde mit Trypsin und α-Chymotrypsin (Protein zu Enzymverhältnis 50:1) bei 37°C 24 Stunden inkubiert. Danach wurde die Probe mit Trichloressigsäure gefällt und zur Auftrennung der erhaltenen proteolytischen Fragmente einer Natriumdodecylsulfat-(SDS)-Polyacrylamidgelektrophorese (PAGE) unterwofen (Lämmli u. Favre, 1973, J. Mol. Biol. 80, 575—599).

Chemische Spaltung

Eine Probe von FMSE-Virus-Glykoprotein wurde mit Trichloressigsäure gefällt, mit Bromcyan versetzt (Bromcyan zu Proteinverhältnis 2:1) und 24 Stunden bei Raumtemperatur im Dunkeln inkubiert. Die erhaltenen Spalt produkte wurde ebenfalls mittels der obigen Natriumdodecylsulfat-Polyacrylamidgeleketrophase Methode getrennt.

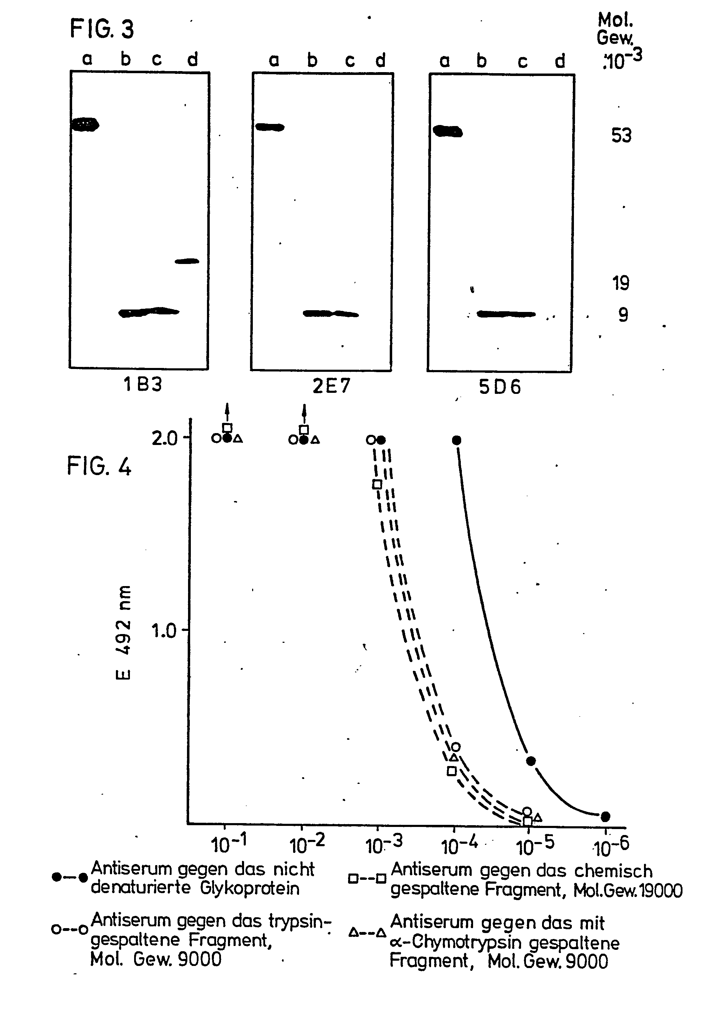

Unter Verwendung der "Immunoblotting"-Technik (Towbin et al., 1979, Proc. Natl. Acad.Sci.U.S.A. 76, 4350—4354) wurden sodann jene durch enzymatische bzw. chemische Spaltung erhaltenen Fragmente identifizert, die mit den vorstehend beschriebenen monoklonalen Antikörpern reagieren. Dabei wurden die aufgetrennten Fragmente zunächst elektrophoretisch auf Nitrocellulose transferiet, die zur Absättigung unspezifischer Bindungsstellen mit 1 %iger Rinderalbuminlösung über Nacht bei Zimmertemperatur inkubiert wurde. Sodann erfolgte die Inkubation mit den beschriebenen monoklonalen Antikörpern, die mit vorhandenen Antigendeterminanten in den aufgetrennten Peptiden reagieren können. Der Nachweis der Bindung monoklonaler Antikörper an einzelne Peptidbanden erfolgte durch weitere Inkubation mit [125]J-markierten Antikörpern gegen Maus-Immunglobuline oder mit [125]J-markiertem Protein A und

6

anschließender Autoradiographie. Das Ergebnis unter Verwendung der protektiven monoklonalen Antikörper 1B3, 2E7 und 5D6 ist schematisch in Fig. 3 dargestellt, wobei der linke Teil der Figur ein Autoradiogramm des Glykoproteins und der Fragmente nach Immundetektion durch den Antikörper 1B3, der mittlere Teil ein Autoradiogramm des Glykoproteins und der Fragmente nach Immundetektion durch den Antikörper 2E7 und der rechte Teil ein Autoradiaogramm des Glykoproteins und der Fragmente nach Immundetektion durch den Antikörper 5D6 wiedergeben. In den vertikalen Spalten a, b, c und d wurden jeweils folgende Proben aufgetrennt:

a) ungespaltenes Glykoprotein

b) Glykoproteinfragmente nach Proteolyse mit Trypsin

c) Glykoproteinfragmente nach Proteolyse mit α-Chymotrypsin

d) Glykoproteinfragmente nach Spaltung mit Bromcyan. Weiters sind rechts in der Figur die zugehörigen Molekulargewichte angegeben.

Es ist aus den Autoradiogrammen ersichtlich, daß alle angeführten Antikörper sowohl mit dem ungespaltenen Glykoprotein E. Mol.Gew. 53.000, reagieren als auch mit einem nach Trypsin und α-Chymotrypsinspaltung erhaltenen Fragment mit einem Mol.Gew. von etwa 9.000. Der Antikörper 1B3 reagiert darüber hinaus auch mit einem nach chemischer Spaltung mit Bromcyan erhaltenen Fragment (Mol.Gew. 19.000). Diese Fragmente enthalten daher Antigen-Determinanten, die mit protektiven monoklonalen Antikörpern reagieren. Diese Fragmente sind deshalb als essentieller Bestandteil einer Vakzine geeignet.

Im folgenden Beispiel 2 wird erläutert, daß die nach Beispiel 1 durch enzymatische oder chemische Spaltung erhaltenen Fragmente eine immunogene Wirkung aufweisen.

Beispiel 2

Die nach Trypsin- bzw. α-Chymotrypsin-Spaltung erhaltenen immunreaktiven Fragmente mit einem Molekulargewicht von etwa 9.000 sowie das nach Bromcyanspaltung erhaltene immunreaktive Fragment mit einem Molekulargewicht von etwa 19.000 wurden mit Hilfe von Polyacrylamidgelektrophorese in Gegenart von SDS isoliert un zur Immunisierung von Mäusen verwendet.

Als Kontrolle diente gereinigtes, nicht denaturiertes Glykoprotein, das nach Solubilisierung des Virus mit dem nichtionischen Detergens Triton X 100 gewonnen wurde. Eine erste immunisierung erfolgte mit komplettem Freunds Adjuvans, für zwei zeitere Immunisierungen im Abstand von jeweils einem Monat wurde inkomplettes Freunds Adjuvans verwendet. Zwei Wochen nach der letzten Immuniserung wurden die Seren in einem Enzymimmunassay auf ihren Gehalt an solchen Antikörpern untersucht, die mit FSME-Virus reagierten.

Die Ergebnisse sind aus Fig. 4 ersichtlich, wobei auf der Abszisse die Serumverdünnung und auf der Ordinate die Extinktion des Testsubstrates (Orthophenylendiamin) angegeben sind, u.zw für das Antiserum gegen das nicht denaturierte Glykoprotein, für das Antiserum gegen das trypsingespaltene Fragment mit dem Molekulargewicht 9.00, für das Antiserum gegen das chemisch gespaltene Fragment mit einem Molekulargewicht von 19.000 und für das Antiserum gegen das mit α-Chymotrypsin gespaltene Fragment mit einem Molekulargewicht von 9.00. Daraus geht hervor, daß die zur Immunisierung verwendeten Fragmente immunogen sind und solche Antikörper induzieren, die mit dem nativen Glykoprotein als Bestandteil der Virushülle reagieren.

In der Tabelle 4 ist die Reaktivität der vorstehend beschriebenen Antisera im Hämagglutinationshemmungstest sowie im Neutralisationstest angegeben. Wie daraus hervorgeht, induzierten die durch die protektiven monoklonalen Antikörper selektionierten Peptide nach Immunisierung von Mäusen sowohol hämagglutinationshemmende als auch virusneutralisierende Antikörper.

Die Herstellung der Peptide bzw. peptidhältigen Verbindungen, die eine oder mehrere Antigendeterminanten des FSME-Virus-Glykoproteins enthalten, kann nicht nur, wie beschrieben, durch enzymatische oder chemische Spaltung des FSME-Virus-Glykoprotein-Moleküls erfolgen, sondern auch durch synthetischen Aufbau aus den Aminosäureresten des Glykoproteinmoleküls mit Hilfe der in er Peptidchemie bekannten Kupplungsreaktionen, wobei die Identifizierung jeweils in der beschriebenen Weise durch die monoklonale Antikörper enthaltenden hochspezifischen reagenzien vorgenommen wird.

Tabelle 1: Reaktivität von FSME-Virus-Glykoprotein-reaktiven monoklonalen
Antikörpern im Enzymimmunassay mit FSME-Virus sowie serologisch
verwandten. Viren.

| Bezeichnung der monoklonalen Antikörper | FSME-Virus | Fernöstl. Subtyp (Sofyn) | Louping Ill Virus | West Nile Virus | Murray Valley encephalitis Virus |
|---|---|---|---|---|---|
| 1C4 | + | +/-[a] | +[b] | -[c] | - |
| 6E2 | + | ÷ | + | + | + |
| 1B3 | + | + | + | - | - |
| 2B6 | + | + | + | +/- | +/- |
| 4D9 | + | +/- | + | - | - |
| 1G2 | + | + | +/- | - | - |
| 2E7 | + | + | + | - | - |
| 5D6 | + | + | + | - | - |

[a] +/- = abgeschwächte Reaktivität im Vergleich zu FSME-Virus

[b] + = gleiche Reaktivität wie mit FSME-Virus

[c] - = fehlende Reaktivität

Tabelle 2: Hämagglutinationshemmungstest (HHT),
Neutralisationstest (NT) und passiver
Mäuseschutzversuch mit monoklonalen
Antikörpern gegen das Struktur-Glykoprotein des FSME-Virus

| Monoklo-naler Antikörper | Titer im HHT | Titer im NT | Titer im passiven Mäuseschutz-versuch |
|---|---|---|---|
| 1C4 | $<$ 10 | $<$ 10 | $<$ 4[a] |
| 6E2 | 1280 | $<$ 10 | $<$ 4 |
| 1B3 | 20 | 10 | 4 |
| 2B6 | 320 | $<$ 10 | $<$ 4 |
| 4D9 | 640 | 100 | 90 |
| 1G2 | $<$ 10 | $<$ 10 | $<$ 4 |
| 2E7 | 160 | 100 | 62 |
| 5D6 | 80/160 | 100 | 15 |

a = weniger als 4 Überlebende bei einer
Verdünnung von 1 : 4

Tabelle 3: Radioimmunassay zum Nachweis unabhängiger oder überlappender Bindungsstellen von monoklonalen Antikörpern am Struktur-Glykoprotein des FSME-Virus

$^{125}$J-markierter Antikörper

| nichtmark. Antikörper | 1C4 | 6E2 | 1B3 | 2B6 | 4D9 | 1G2 | 2E7 | 5D6 |
|---|---|---|---|---|---|---|---|---|
| 1C4 | +[a] | −[b] | − | − | − | − | − | − |
| 6E2 | − | + | − | + | − | − | − | − |
| 1B3 | − | − | + | − | − | − | + | + |
| 2B6 | − | + | − | + | + | − | − | − |
| 4D9 | − | − | − | + | + | − | − | − |
| 1G2 | − | − | − | − | − | + | + | − |
| 2E7 | − | − | + | − | − | + | + | + |
| 5D6 | − | − | + | − | − | − | + | + |

[a] + = Blockierung des $^{125}$J-markierten Antikörpers durch den nichtmarkierten Antikörper

[b] − = Keine Blockierung

# EP 0 106 837 B1

### Tabelle 4: Funktionelle Charakterisierung von nach Immunisierung von Mäusen mit Fragmenten des FSME-Virus-Glykoproteins erhaltenen Antiseren

| | Antikörper Titer | |
|---|---|---|
| | $HHT^a$ | $NT^b$ |
| Antiserum gegen nicht de-naturiertes Glykoprotein | 640 / 1280 | 256 |
| Antiserum gegen nach Trypsin-spaltung erhaltenes Fragment (Mol.Gew. 9.000) | 40 | 4 |
| Antiserum gegen nach $\alpha$-Chymo-trypsinspaltung erhaltenes Fragment (Mol.Gew. 9.000) | 40 | 4 |
| Antiserum gegen nach chemi-scher Spaltung durch Brom-cyan erhaltenes Fragment (Mol.Gew. 19.000) | 20 | 4 |

[a]HHT: Hämagglutinationshemmungstest
[b]NT : Neutralisationstest

## Patentansprüche

1. Verwendung von Peptiden entsprechend einer Teilaminosäuresequenz des FSME-Virus-Glykoproteins E zur Herstellung von gegen FSME-Virusinfektionen wirksamen Vakzinen, enthaltend eine oder mehrere Antigendeterminanten des FSME-Virus, gekennzeichnet durch ihre Reaktivität gegenüber einem der monoklonalen Antikörper 4D9, 1B3, 5D6, 2E7 gemäß Fig. 1, die definiert sind durch
    a) protective Wirkung im passiven Mäuseschutzversuch und/oder
    b) virusneutralisierende Wirkung im Neutralisationstest.

2. Verwendung von Peptiden gemäß Anspruch 1 mit Reaktivität gegenüber dem Antikörper 1B3, der durch die weiteren Merkmale
    c) eine fehlende oder sehr niedrige Aktivität im Hämagglutinationshemmungstest,
    d) gleiche Reaktivität im Enzymimmunassay mit FSME-Virus, dem fernöstlichen Subtyp des FSME-Virus (Sofyn) und Louping III Virus; fehlende Reaktivität mit West Nile und Murray Valley Encephalitis Virus,
    e) Hemmung der Bindung monoklonaler Antikörper der Charakteristik 2E7 und 5D6 (Fig. 1) an das Virus gemessen im kompetitiven Radioimmunassay definiert ist.

3. Verwendung von Peptiden gemäß Anspruch 1 mit Reaktivität gegenüber dem Antikörper 4D9, der durch die weiteren Merkmale
    f) eine hämagglutinationshemmende Aktivität,
    g) gleiche Reaktivität im Enzymimmunassay mit FSME-Virus und Louping III Virus, verminderte Reaktivität mit dem fernöstlichen Subtyp des FSME-Virus (Sofyn), fehlende Reaktivität mit West Nile und Murray Valley Encephalitis Virus,
    h) Hemmung der Bindung monoklonaler Antikörper der Charakteristik 2B6 (Fig. 1) an das Virus gemessen im kompetitiven Radioimmunassay definiert ist.

11

EP 0 106 837 B1

4. Verwendung von Peptiden gemäß Anspruch 1 mit Reaktivität gegenüber dem Antikörper 2E7, der durch die weiteren Merkmale

f) eine hämagglutinationshemmende Aktivität,

d) gleiche Reaktivität im Enzymimmunassay mit FSME-Virus, dem fernöstlichen Subtyp des FSME-Virus (Sofyn) und Louping III Virus; fehlende Reaktivität mit West Nile und Murray Valley Encephalitis Virus,

i) Hemmung der Bindung monoklonaler Antikörper der Charakteristik 5D6, 1B3 und 1G2 (Fig. 1), gemessen im kompetitiven Radioimmunassay, definiert ist.

5. Verwendung von Peptiden gemäß Anspruch 1 mit Reaktivität gegenüber dem Antikörper 5D6, der durch die weiteren Merkmale

f) eine hämagglutinationshemmende Aktivität,

d) gleiche Reaktivität im Enzymimmunassay mit FSME-Virus, dem fernöstlichen Subtyp des FSME-Virus (Sofyn) und Louping III Virus; fehlende Reaktivität mit West Nile und Murray Valley Encephalitis Virus,

k) Hemmung der Bindung monoklonaler Antikörper der Charakteristik 1B3 und 2E7 (Fig. 1), gemessen im kompetitiven Radioimmunassay, definiert ist.

6. Verwendung von Peptiden gemäß den Ansprüchen 1 bis 5 mit den weiteren Kennzeichen, daß sie frei sind von Sequenzen die, mit blockierenden monoklonalen Antikörpen 2B6 bzw. 1G2 (Fig. 1) folgender Charakteristik reagieren, wobei der monoklonale Antikörper 2B6 definiert ist durch

l) Hemmung der Bindung des protektiven monoklonalen Antikörpers 4D9 sowie des nichtprotektiven monoklonalen Antikörpers 6E2, gemessen im kompetitiven Radioimmunassay,

m) fehlende oder sehr geringe Aktivität im passiven Mäuseschutzversuch,

n) fehlende oder sehr niedrige Aktivität im Neutralisationstest,

f) hämagglutinationshemmende Aktivität,

d) gleiche Reaktivität im Enzymimmunassy mit FSME-Virus, dem fernöstlichen Subtyp des FSME-Virus (Sofyn) und Louping III Virus, abgeschwächte Reaktivität mit West Nile und Murray Valley Encephalitis Virus, und der monoklonale Antikörper 1G2 definiert ist durch

o) Hemmung der Bindung protektiver monoklonaler Antikörper des Charakteristik 2E7, gemessen im kompetitiven Radioimmunassay,

m) fehlende oder sehr geringe Aktivität im passiven Mäuseschutzversuch,

n) fehlender oder sehr niedrige Aktivität im Neutralisationstest,

c) fehlende oder sehr geringe Aktivität im Hämagglutinationshemmungstest,

p) gleiche Reaktivität im Enzymimmunassay mit FSME-Virus und dem fernöstlichen Subtyp des FSME-Virus (Sofyn), abgeschwächte Reaktivität mit Louping III Virus, fehlende Reaktivität mit West Nile und Murray Valley Encephalitis Virus.

**Revendications**

1. Utilisation de peptides correspondant à une séquence partielle d'aminoacides de la glucoprotéine E du virus de la méningo-encéphalite du début de l'été (FSME) pour la fabrication de vaccins efficaces contre les infections à virus FSME, contenant un ou plusieurs déterminants antigéniques du virus FSME, caractérisés par leur réactivité vis-à-vis de l'un des anticorps monoclonaux 4D9, 1B3, 5D6, 2E7 selon la figure 1, qui sont définis par

a) une action protectice dans l'essai de protection passive des souris et/ou,

b) une action neutralisante du virus dans le test de neutralisation.

2. Utilisation de peptides selon la revendication 1 doués de réactivité vis-à-vis de l'anticorps 1B3, qui est défini par les autres caractéristiques suivantes:

c) l'absence d'activité ou une activité très faible dans le test d'inhibition de l'hémagglutination,

d) la même réactivité dans l'essai immuno-enzymatique avec le virus FSME, le sous-type extrême-oriental du virus FSME (Sofyn) et le virus Louping III; pas de réactivité avec le virus West-Nile et le virus de L'encéphalite de Murray Valley,

e) inhibition de la fixation d'anticorps monoclonaux de caractéristiques 2E7 et 5D6 (figure 1) sur le virus mesurée dans l'essai radio-immunologique compétitif.

3. Utilisation de peptides selon la revendication 1 doués de réactivité vis-à-vis de l'anticorps 4D9, qui est défini par les autres caractéristiques suivantes:

f) une activité inhibitrice de l'hémagglutination,

g) la même réactivité dans l'essai immuno-enzymatique avec le virus FSME et le virus Louping III; réactivité réduite avec le sous-type extrême-oriental du virus FSME (Sofyn); pas de réactivité avec le virus West-Nile et le virus de l'encéphalite de Murray Valley,

h) inhibition de la fixation d'anticorps monoclonaux de caractéristique 2B6 (figure 1) sur le virus, mesurée dans l'essai radio-immunologique compétitif.

4. Utilisation de peptides selon la revendication 1 doués de réactivité vis-à-vis de l'anticorps 2E7, qui est défini par les autres caractéristiques suivantes:

f) une activité inhibitrice de l'hémagglutination,

d) la même réactivité dans l'essai immuno-enzymatique avec le virus FSME, le sous-type extrême-

12

oriental du virus FSME (Sofyn) et le virus Louping III; pas de réactivité avec le virus West-Nile et le virus de l'encéphalite de Murray Valley,

i) inhibition de la fixation de l'anticorps monoclonaux de caractéristiques 5D6, 1B3 et 1G2 (figure 1) mesurée dans l'essai radio-immunologique compétitif.

5. Utilisation de peptides selon la revendication 1 doués de réactivité vis-à-vis de l'anticorps 5D6, qui est défini par les autres caractéristiques suivantes:

f) une activité inhibitrice de l'hémagglutination,

d) la même réactivité dans l'essai immuno-enzymatique avec le virus FSME, le sous-type extrême-oriental du virus FSME (Sofyn) et le virus Louping III; pas de réactivité avec le virus West-Nile et le virus de l'encéphalite de Murray Valley,

k) inhibition de la fixation d'anticorps monoclonaux de caractéristiques 1B3 et 2E7 (figure 1), measurée dans l'essai radio-immunologique compétitif.

6. Utilisation de peptides selon les revendications 1 à 5 ayant les caractéristiques supplémentaires qu'ils sont exempts de séquences qui réagissent avec les anticorps monoclonaux bloquants 2B6 ou 1G2 (figure 1) de caractéristiques suivantes, l'anticorps monoclonal 2B6 étant défini par

l) l'inhibition de la fixation de l'anticorps monoclonal protecteur 4D9 ainsi que de l'anticorps monoclonal non protecteur 6E2, mesurée dans l'essai radio-immunologique compétitif,

m) pas d'activité ou une très faible activité dans l'essai de protection passive des souris,

n) pas d'activité ou une très faible activité dans le test de neutralisation,

f) une activité inhibitrice de l'hémagglutination,

d) la même réactivité dans l'essai immuno-enzymatique avec le virus FSME, le sous-type extrême-oriental du virus FSME (Sofyn) et le virus Louping III; réactivité atténuée avec le virus West-Nile et le virus de l'encéphalite Murray Valley,

et l'anticorps monoclonal 1G2 étant défini par

o) l'inhibition de la fixation des anticorps monoclonaux protecteur de caractéristiques 2E7, mesurée dans l'essai radioimmunologique compétitif,

m) pas d'activité ou une très faible activité dans l'essai de protection passive des souris,

n) pas d'activité ou une très faible activité dans le test de neutralisation,

c) pas d'activité ou une très faible activité dans le test d'inhibition de l'hémagglutination,

p) la même réactivité dans l'essai immuno-enzymatique avec le virus FSME et le sous-type extrême-oriental du virus FSME (Sofyn); une réactivité atténuée vec le virus Louping III; pas de réactivitié avec le virus West-Nile et le virus de l'encéphalite Murray Valley.

**Claims**

1. Use of peptides corresponding to a partial aminoacid sequence of the TBE-virus-glycoprotein E for producing vaccines active against TBE virus infections, containing one or more antigen determinants of the TBE virus, characterised by their reactivity relative to one of the monoclonal antibodies 4D9, 1B3, 5D6, 2E7 according to Fig. 1, which are defined by

a) protective effect in the passive mouse protection test and/or

b) virus-neutralising effect in the neutralisation test.

2. Use of peptides according to claim 1 with reactivity relative to the antibody 1B3, which is defined by the further features

c) a missing or very low activity in the hemagglutionation inhibition test,

d) equal reactivity in the enzyme immunoassay with TBE virus, the Far-Eastern sub-type of TBE virus (Sofyn) and Louping III virus; missing reactivity with West Nile and Murray Valley Encephalitis virus,

e) inhibition of binding monoclonal antibodies of the characteristic 2E7 and 5D6 (Fig. 1) to the virus, measured in the competitive radioimmunoassay.

3. Use of peptides according to claim 1 with reactivity relative to the antibody 4D9, which is defined by the further features

f) a hemagglutination-inhibiting activity,

g) equal reactivity in the enzyme immunoassay with TBE virus and Louping III virus, reduced reactivity with the Far-Eastern sub-type of TBE virus (Sofyn), missing reactivity with the West Nile and Murray Valley Encephalitis virus,

h) inhibition of binding monolonal antibodies of the characteristic 2B6 (Fig. 1) to the virus, measured in the competitive radioimmunoassay.

4. Use of peptides according to claim 1 with reactivity relative to the antibody 2E7, which is defined by the further features

f) a hemagglutination-inhibiting activity,

d) equal reactivity in the enzyme immunoassay with TBE virus, the Far-Eastern sub-type of TBE virus (Sofyn) and Louping III virus; missing reactivity with West Nile and Murray Valley Encephalitis virus,

i) inhibition of binding monoclonal antibodies of the characteristic 5D6, 1B3 and 1G2 (Fig. 1), measured in the competitive radioimmunoassay.

5. Use of peptides according to claim 1 with reactivity relative to the antibody 5D6, which is defined by the further features

f) a hemagglutination-inhibiting activity,

d) equal reactivity in the enzyme immunoassay with TBE virus, the Far-Eastern sub-type of TBE virus (Sofyn) and Louping III virus; missing reactivity with the West Nile and Murray Valley Encephalitis virus,

k) inhibition of binding monoclonal antibodies of the characteristic 1B3 and 2E7 (Fig. 1), measured in the competitive radioimmunoassay.

6. Use of peptides according to claims 1 to 5 with the further features that they are free from sequences reacting with blocking monoclonal antibodies 2B6 and 1G2, respectively, (Fig. 1) of the following characteristic, wherein the monoclonal antibody 2B6 is defined by

l) inhibition of binding the protective monoclonal antiboody 4D9 as well as of the non-protective monoclonal antibody 6E2, measured in the competitive radioimmunoassay,

m) missing or very low activity in the passive mouse protection test,

n) missing or very low activity in the neutralisation test,

f) a hemagglutination-inhibiting activity,

d) equal reactivity in the enzyme immunoassay with TBE virus, the Far-Eastern sub-type of TBE virus (Sofyn), and Louping III virus; reduced reactivity with West Nile and Murray Valley Encephalitis virus, and the monoclonal antibody 1G2 is defined by

o) inhibition of binding protective monoclonal antiboodies of the characteristic 2E7, measured in the competitive radioimmunoassay,

m) missing or very low activity in the passive mouse protection test,

n) missing or very low activity in the neutralisation test,

c) missing or very low activity in the hemagglutination-inhibition test,

p) equal reactivity in the enzyme immunoassay with TBE virus and the Far-Eastern sub-type of TBE virus (Sofyn) reduced reactivity with the Louping III virus, missing reactivity with the West Nile and Murray Valley Encephalitis virus.

## FIG.1

FIG.2

α - Chymotrypsin - Behandlung

1B3    4D9    2E7    5D6

Trypsin - Behandlung

% Blockierung

0  20  40  60  80  100

40  4  0,4  0,04   µg/ml

——— Peptide des FSME-Virus Glykoproteins

——○—— Peptide von Rinderalbumin

FIG. 3

FIG. 4

●—● Antiserum gegen das nicht denaturierte Glykoprotein

□--□ Antiserum gegen das chemisch gespaltene Fragment, Mol.Gew.19000

○--○ Antiserum gegen das trypsin-gespaltene Fragment, Mol. Gew. 9000

△--△ Antiserum gegen das mit α-Chymotrypsin gespaltene Fragment, Mol.Gew. 9000